(19) Europäisches Patentamt

European Patent Office

Office européen des brevets

(11) **EP 1 202 785 B1**

(12) **EUROPÄISCHE PATENTSCHRIFT**

(45) Veröffentlichungstag und Bekanntmachung des Hinweises auf die Patenterteilung:
**14.05.2003 Patentblatt 2003/20**

(21) Anmeldenummer: **00956199.4**

(22) Anmeldetag: **18.07.2000**

(51) Int Cl.7: **B01D 15/08**

(86) Internationale Anmeldenummer:
**PCT/EP00/06853**

(87) Internationale Veröffentlichungsnummer:
**WO 01/007137 (01.02.2001 Gazette 2001/05)**

(54) **SÄULENCHROMATOGRAPHISCHES TRENNVERFAHREN**

COLUMN CHROMATOGRAPHY SEPARATION METHOD

PROCEDE DE SEPARATION PAR CHROMATOGRAPHIE SUR COLONNE

(84) Benannte Vertragsstaaten:
**AT BE CH CY DE DK ES FI FR GB GR IE IT LI LU MC NL PT SE**

(30) Priorität: **21.07.1999 DE 19934168**

(43) Veröffentlichungstag der Anmeldung:
**08.05.2002 Patentblatt 2002/19**

(73) Patentinhaber: **KD Pharma Bexbach GmbH**
**66450 Bexbach (DE)**

(72) Erfinder:
• **KRUMBHOLZ, Rudolf**
**F-57510 Holving (FR)**
• **SCHIRRA, Norbert**
**D-66333 Völklingen (DE)**
• **TREITZ, Manfred**
**D-66352 Grossrosseln (DE)**

(74) Vertreter: **Ackermann, Joachim, Dr.**
**Postfach 11 13 26**
**60048 Frankfurt am Main (DE)**

(56) Entgegenhaltungen:
**EP-A- 0 127 926** **US-A- 5 422 007**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**EP 1 202 785 B1**

**Beschreibung**

Die vorliegende Erfindung betrifft ein säulenchromatographisches Trennverfahren unter Verwendung eines flüssigen und niedermolekularen Fluids als Eluent zur Isolierung und/oder Reinigung und/oder präparativen Gewinnung von (Natur)Stoffen aus Stoffgemischen, vorzugsweise Naturstorfgemischen.

Säulenchromatographische Trennverfahren spielen vermehrt in der Rohstoff-Aufbereitung, in der Produktreinigung, in der chemische Synthese sowohl im Laboratoriumsmaßstab wie auch bei großtechnischen Produktionen eine bedeutende Rolle. Ebenfalls in der chemischen Analytik sind sie für die Isolierung, Identifikation und quantitativer / qualitativer Bestimmung einzelner Komponenten in Substanzgemischen unentbehrlich. Weitere wichtige Anwendungsgebiete für derartige Trennungen sind das Recycling, wobei aus Altmaterialien, wie Abwasser und Abgasen Gifte und Schadstoffe, aber auch Wertstoffe abgeschieden werden.

Aus Kostengründen haben es bisher jedoch nur wenige säutenchromatographische Trennverfahren bis zur kommerziellen Nutzung gebracht. Insbesondere im Rahmen der präparativen Rohstoffgewinnung, insbesondere aus Naturstoffgemischen - sowie deren Analytik - kommen säulenchromatographische Trennverfahren, wie HPLC ("High Performance Liquid Chromatography") oder Hochleistungs- bzw. Hochdruck-Flüssigkeitschromatographie sowie SFC ("Supercritical Fluid Chromatograhie") zum Einsatz.

Die HPLC macht sich die Erkenntnis zunutze, daß die Trennleistung einer Säule mit abnehmender Korngröße der stationären Phase zunimmt. Bei der HPLC wird mit erheblich feinerem chromatographischen Trennmaterial (3-10 μm) als bei der Gelchromatographie (39-75 μm) oder der "klassischen" Säulenchromatographie (120-200 μm) gearbeitet. Die Feinteiligkeit der Trennmaterialien erfordert allerdings die Anwendung hoher Drücke (bis zu 40 MPa), was mit erheblichem technischen Aufwand verbunden ist. Die Säulen sind üblicherweise zwischen 5 - 100 cm (zumeist jedoch bis 25 cm) lang mit einem Innendurchmesser von 1-25 μm. Mit Kieselgel oder ähnlich porösem Material (von 10 μm oder weniger) gefüllt, kann eine 25 cm lange Säule 5000 Böden (theoretische Trennstufen) aufweisen - selbst Bodenzahlen von 65000/m können je nach stationärer Phase erzielt werden. Typische HPLC-Trennungen werden bei Raumtemperatur, vorzugsweise bei 20 bis 40°C, durchgeführt (Fa. Merck, Chrombook, 1996, Darmstadt, Fa. Phenomenex, Chromatographiekatalog, 99/00, Torrance, CA, USA). Die notwendigen Pumpen können in der analytischen HPLC bis zu einem Druck von 400 bar betrieben werden. In der präparativen HPLC werden Pumpen betrieben bis zu einem Druck von 70 (Pumpenleistung: 300 l/h) bis 100 bar (Pumpenleistung: 60 l/h Eluent (Flüssigkeit)) (vgl. z.B. Fa. Merck, Chrombook, S. 233, 1996, Darmstadt). Der limitierende Faktor bei der HPLC ist, insbesondere bei hohen Flüssen, der Pumpendruck. Die verwendeten Säulenlängen sind daher zumeist kurz; es gilt: je kleiner der Teilchendurchmesser des verwendeten chromatographischen Materials, desto kürzer müssen die Säulen werden. Prinzipiell kann eine Kompensation durch höhere maximale Arbeitsdrücke erfolgen, dies erhöht jedoch die Betriebskosten enorm und kann das HPLC - Verfahren im Rahmen einer präparativen Anwendung in die Ünwirtschaftlichkeit führen.

Die SFC hat sich als säulenchromatographisches Trennverfahren mit Gasen ($CO_2$, $N_2O$, $SF_6$ etc.) im überkritischen Zustand als mobile Phase durchgesetzt. Bei der SFC werden Flüssigkeiten und Gase unter Druck erhitzt, wobei diese oberhalb der jeweiligen kritischen Temperatur und des kritischen Drucks in den überkritischen Zustand überführt werden. In diesem Zustand zeichnen sich überkritische Flüssigkeiten gegenüber echten Flüssigkeiten nicht nur durch ihre geringere Dichte, viel niedrigere Viskosität und viel höhere Diffusionskoeffizienten aus, sondern vor allem durch ihr hervorragendes Lösungsvermögen. Deshalb finden überkritische Flüssigkeiten aus $CO_2$, Ethylen, Propan, Ammoniak, Distickstoffdioxid, Wasser, Toluol, Stickstoffheterocyclen u. a. Anwendung in der Extraktion, der sogenannten Supercritical Fluid Extraction (SFE od. SCFE; auch "Destraktion" genannt) - von Naturstoffen. Bekannt sind z. B. die Extraktion von Coffein aus Kaffee durch überkritisches $CO_2$, von Hopfenbitterstoffen, Riechstoffen, von Kohlenwasserstoffen aus Erdöl oder Kohle, zur Reaktivierung von Heterogenkatalysatoren (Vgl. Wenclawiak (Hrsg.), Analysis with Supercritical Fluids: Extraction and Chromatography, Berlin: Springer 1992).

In der Regel wird in der SFC bei Temperaturen über 35°C und einem Druck auf der Säulenauslaßseite von über 120 bar gearbeitet.

Beschrieben sind zahlreiche Trennungen bei Temperaturen unterhalb der kritischen Temperatur der verwendeten mobilen Phase; vgl. K. Anton, C. Berger (Ed.); Packed Columns, Marcel Dekker Inc., New York, Basel, Hongkong, 1998.

Die Trennung von Stoffgemischen, insbesondere von Naturstoffgemischen, mittels SFC wird in einer Reihe von Publikationen beschrieben, wie in JP-A-10/316,991; Chirality (1992); 4(4), 252-62; J. chromatogr. (1989), 464(1), 125-37; Nihon Yukagakkaishi (1997), 46(11), 1335-1345; Semin. Food Anal. (1996), 1(2), 101-116; J. High Resolut. Chromatogr. (1996), 19(10), 569-570; Jasco Rep. (1991), 33(1), 6-11; Anal. Chem. (1990), 62(12), 394R-402R; und J. Chromatogr. Sci. (1990), 28(1), 9-14.

Vérillon und Coleman beschreiben auf Seite 64 (Anton supra) den Temperatur- und Druckbereich für das Arbeiten mit Fluiden- wie $CO_2$ - zwischen 3 bis 200°C und 90 - 400 bar. Die Trennungen erfolgen im überkritischen und subkritischen Bereich, jedoch nicht im flüssigen Bereich, d.h in einem Druckbereich unter dem kritischen Druck und Temperatur.

Jusforques beschreibt auf S. 409 in Kap. 14 (supra Anton) den erforderlichen Arbeitsbereich mit Temperaturen zwischen 0 und 150°C und Drücken zwischen 100 und 350 bar an (SFC-Apparatur: Super C12 im Rahmen der präparativen SFC).

Die Firma Prochrom gibt ebenfalls einen Arbeitsdruckbereich zwischen 100 und 350 bar an, bei Temperaturen zwischen 10 und 80°C (Fa. Prochrom, Prospekt zu semipreparative Supercritical Fluid Chromatograph, Type: SuperC 20, 1998, Chamigneulle, Frankreich).

EP 0 099 765 B1 offenbart, daß der Einlaßdruck des Gemisches in die Säule größer als der kritische Druck des Eluenten sein muß, und zwar zwischen 1,05 und 3. Drücke kleiner als der kritische Druck werden ausdrücklich als Abscheidebedingungen beschrieben.

EP-A-0 127 926 offenbart ein Chromatographieverfahren, bei dem verflüssigte Gase als Eluenten verwendet werden. Die Gase werden unterhalb der kritischen Temperatur belassen, werden jedoch durch Kompression verflüssigt, wobei überkritische Drücke zum Einsatz kommen.

M. Perrut beschreibt in NO 163139 als Trennbedingungen nur den überkritischen und subkritischen Bereich und zwar Drücke von 70-250 bar und Temperaturen zwischen 25 und 80°C.

Im Stand der Technik sind keine chromatographischen Trennungen realisiert für mobile Phasen, mit einem Arbeitsbereich unter der kritischen Temperatur und Druck.

Schuitz et. al (Schultz, W.G.; Randall, J.M.: Food Technol. 24, 1282 (1970)) beschreibt lediglich eine unspezifische Extraktion bei 22°C und 63 bar von Aromakonzentraten aus homogenisierten Früchten und Fruchtsäften. Eine chromatographische Trennung ist nicht erfolgt.

Der vorliegenden Erfindung liegt somit die Aufgabe zugrunde, ein säulenchromatographisches Verfahren im Rahmen einer Flüssigkeitschromatographie für die großtechnische Isolierung und / oder Reinigung und / oder präparative Gewinnung von (Natur)Stoffen aus (Natur)Stoffgemischen bereitzustellen, das aufgrund einer hohen Trennleistung zu reinen Produkten führt.

Somit betrifft die vorliegende Erfindung ein Verfahren zur säutenchromatographischen Trennung von Stoffgemischen, wobei als Eluent ein Fluid oder eine Mischung von Fluiden in flüssigem, nicht-subkritischem und nicht-kritischem Zustand (T < $T_{kr}$ und p < $p_{kr}$) zum Einsatz kommt, die bei 25°C und 1 bar gasförmig sind (nachstehend erfindungsgemäßes Verfahren).

Bei den erfindungsgemäß zum Einsatz kommenden Eluenten handelt es sich um niedermolekulare Verbindungen, die bei Normalbedingungen (25°C; 1 bar) gasförmig sind und die unter derartigen Temperatur- und Druckbedingungen zum Einsatz kommen, daß sie im flüssigen Zustand aber nicht im kritischen Zustand oder nicht im subkritischen Zustand (also nicht oberhalb der kritischen Temperatur und unterhalb des kritischen Druckes) vorliegen.

Bevorzugte Eluenten für das erfindungsgemäße Verfahren sind leichtflüchtige Verbindungen mit geringer Wechselwirkung

Besonders bevorzugte Eluenten sind flüssiges Distickstoffoxid ($N_2O$); flüssige Fluoroder Fluorkohlenwasserstoffe ("Freone"), wie z.B. Chlortrifluormethan ($CClF_3$), Trifluormethan ($CHF_3$), Tetrafluormethan ($CF_4$), 1,1,1,2-Tetrafluorethan ($CF_4H_2$), flüssiges Kohlendioxid ($CO_2$), flüssiges Schwefelhexafluorid ($SF_6$), flüssiges Propen ($C_3H_6$), flüssiges Propan ($C_3H_8$), flüssiger Ammoniak ($NH_3$), flüssiges Schwefeldioxid ($SO_2$), flüssiges Xenon (Xe), flüssiges Ethan ($C_2H_8$), welche bei Normalbedingungen gasförmig vorliegen.

Vorzugsweise werden solche Eluenten eingesetzt, die eine dynamische Viskosität von $10^{-4}$ - $10^{-6}$ Pa s, vorzugsweise $2*10^{-4}$ - $2*10^{-6}$ Pa s, eine Dichte von 0,5 - 1,2 g/ml, vorzugsweise 0,5-1,2 g/ml aufweisen.

Die Messung der dynamischen Viskosität erfolgt für die Zwecke dieser Anmeldung mit einem Kapillarviskosimeter bei 25°C.

Im erfindungsgemäßen Verfahren liegen solche niederen Moleküle als Elüent in der mobilen Phase flüssig vor.

Es werden vorzugsweise solche Eluenten eingesetzt, die dem Fachmann auf dem Gebiet der SFC bekannt sind.

Flüssiges Kohlendioxid ist jedoch ganz besonders bevorzugt und zwar in dem flüssigen Temperaturbereich von -57°C bis 31,3°C, vorzugsweise von 0 bis 20°C.

Ferner ist flüssiges Kohlendioxid ganz besonders bevorzugt und zwar in dem Druckbereich von 30 bis 73,7 bar und ganz bevorzugt 30 bis 60 bar.

Bei Kohlendioxid beträgt der kritische Druck 73,7 bar und die kritische Temperatur beträgt 31,3°C. Der Tripelpunkt des Kohlendioxids liegt bei -57°C und 5,2 bar.

Selbstverständlich können die genannten Eluenten auch in einer Mischung vorliegen.

Der Arbeitsbereich des eingesetzten niedermolekularen Eluenten ist die flüssige Phase mit den zugehörigen p,T-Werten.

Diese p,T-Werte herrschen ebenfalls an der gewählten chromatographischen Trennsäule über welche der Eluent als mobile Phase geführt wird.

Säulenchromatographisches Trennverfahren im Sinne dieser Erfindung bedeutet, daß eine Stofftrennung durch Verteilung, also durch den Lösevorgang in beiden, miteinander nicht mischbaren Phasen und durch Adsorption an einem Feststoff (Adsorbens) an einer stationären Phase in Gegenwart eines Eluenten als mobile Phase erfolgt. Insofern kann von einer Flüssig-fest-Chromatographie (engl. Liquid-Solid Chromatography, LSC) gesprochen werden. Zu den hier benutzten Begriffen existieren Terminologie-Vorschläge der IUPAC, auf diese wird sich ausdrücklich bezogen.

In einer besonderen Ausführungsform wird die mobile Phase, enthaltend ein Eluent, mit Hilfe eines in der Chromatographie üblichen Modifiziermittels, wie einem unter Betriebsbedingungen flüssigem Alkohol, z.B. Ethanol oder Methanol, versetzt. Dieses Modifiziermittel kann ggf. mit weiteren Hilf- und Zusatzstoffen, wie Säuren oder Basen (z.B. Essigsäure oder Diethylamin) versetzt werden.

Im Rahmen des erfindungsgemäßen Verfahren ergeben sich im Vergleich zu den bekannten Verfahren, wie HPLC- oder SFC-Verfahren zahlreiche Vorteile.

Besonders vorteilhaft gegenüber den bekannten HPLC- oder SFC-Verfahren ist, daß im erfindungsgemäßen Verfahren geringere Drücke angewendet werden müssen, beispielsweise lediglich Drücke bis ca. 70 bar. Dadurch können Kosten eingespart werden, da alle benötigten Bauteile, wie Pumpen, Druckaufnehmer oder Rohrleitungen, entsprechend ausgelegt bzw. kleiner dimensioniert werden können. Die erforderlichen Sicherheitsanforderungen zur Arbeitssicherheit und Betriebssicherheit können reduziert werden.

Es wurde nunmehr in überraschender Weise gefunden, daß das erfindungsgemäße Verfahren in Gegenwart jedweder handelsüblicher fester stationärer Phasen erfolgen kann, wie sie im Rahmen der HPLC und/oder der SFC verwendet werden.

Bevorzugt sind jedoch Säulen mit modifizierten oder unmodifizierten Phasen auf Basis von Kieselgel, Aluminiumoxid oder Titandioxid sowie geträgerte polymere stationäre Phasen, wie z.B. auf Kieselgel, Aluminiumoxid oder Titandioxid aufgebrachte Diolphase, Aminopropylphase, RP8 und RP18 Phasen, wobei diese Phasen insbesondere auf Kieselgelträgern aufgebracht sind.

Das erfindungsgemäße Verfahren kann für Säulenlängen in weiten Bereichen eingesetzt werden. Üblicherweise sind die Säulen mindestens 10 cm lang, bevorzugt sind jedoch Längen von 0,25 - 2,0 m, besonders bevorzugt ist 1,1 - 1,7 m.

Diese höheren Säulenlängen gewährleisten eine hohe Trennleistung und können erreicht werden, da das erfindungsgemäße Verfahren keinen großen Druckabfall in der Säule verursacht.

Der Druckabfall kann nach Formel 1 an Tabelle 1 abgeschätzt werden. Da die Viskosität der zum Einsatz kommenden flüssigen Eluenten klein gewählt ist, ist auch der resultierende Druckabfall gering. Durch den geringen Druckabfall pro cm Säule kann die Säule bei gleichem Teilchendurchmesser der stationären Phase entsprechend lang gewählt werden oder es können im Vergleich zur HPLC kleinere Teilchendurchmesser bei konstanter Säulenlänge verwendet werden.

Das erfindungsgemäße Verfahren kann mit üblichen Anordnungen betrieben werden, wie sie auch in den bekannten SFC-Verfahren zum Einsatz kommen.

Zur weiteren Erläuterung der Erfindung wird nachfolgend der Betrieb einer üblichen SFC-Anordnung im industriellen Maßstab mit dem Eluenten $CO_2$ beschrieben.

Einzuspeisendes $CO_2$ wird aus einem flüssig $CO_2$-Vorratstank mittels eines Wärmetauschers abgekühlt. Das von der Pumpe geförderte $CO_2$ wird mittels des Wärmetauschers auf überkritische Bedingungen gebracht (T>31°C, p>74bar), typischerweise 40-60°C. Jenes $CO_2$ strömt durch die Trennsäule und wird an einem Druckregelventil entspannt. Dieses Druckregelventil regelt den Säulenauslaßdruck, der am Ende der Trennsäule herrscht und über dem kritischen Druck gehalten (p>74bar) wird. Dadurch werden in der Trennsäule überkritische Trennbedingungen gewährleistet. Das $CO_2$ kühlt sich durch die adiabatische Expansion am Druckregelventil ab. Anschließend wird das $CO_2$ mit einem Wärmetauscher aufgeheizt und in den gasförmigen Zustand gebracht. In einem Abscheider wird das Produkt vom gasförmigen $CO_2$ getrennt. Das gasförmige $CO_2$ gelangt über den Wärmetauscher, der es wieder verflüssigt, in den Vorratstank. Es ergibt sich ein geschlossener $CO_2$-Kreislauf. Das Produkt wird aus dem Vorratsgefäß mit Hilfe der Pumpe diskontinuierlich auf die Trennsäule injiziert. Für diese übliche SFC-Anordnung sind energieintensiv total mindestens vier Wärmetauscher notwendig.

Im Rahmen des erfindungsgemäßen Verfahren ergeben sich zusätzlich zu der oben für das SFC-Verfahren beschriebenen Vorgehensweise zahlreiche Möglichkeiten einer funktionalen Anordnung von denen einige nachfolgend beschrieben werden.

Der besondere Vorteil des erfindungsgemäßen Verfahrens liegt darin, daß ein energieaufwendiges wechselndes Abkühlen und Erhitzen des $CO_2$ vermieden wird.

Einzuspeisendes $CO_2$ wird aus einem flüssig $CO_2$ -Vorratstank mittels eines Wärmetauschers abgekühlt. Das von der Pumpe geförderte $CO_2$ wird mittels des Wärmetauschers auf die Trenntemperatur eingestellt. Dieser kann jedoch sehr klein sein, da das $CO_2$ nur von typischerweise 0-5°C auf 5-20°C erwärmt wird.

Werden niedrige Trenntemperaturen, z.B. 5°C, gewählt, so kann dieser Wärmetauscher zudem außer Betrieb genommen werden. Das flüssige $CO_2$ strömt durch die Trennsäule. Mit Hilfe eines Umschaltventiles werden die einzelnen Fraktionen auf sogenannte Fraktionierungssäulen umgeleitet, beispielsweise auf drei Fraktionierungssäulen.

Es sind beliebig viele Fraktionierungssäulen möglich, im einfachsten Fall jedoch eine Fraktionierungssäule und eine zusätzliche Rohrleitung, welches das Umschaltventil mit einem weiteren Umschaltventil verbindet.

Der Säulenauslaßdruck wird mit einem Druckregelventil geregelt. An diesem Ventil erfolgt die Entspannung des $CO_2$. Nach Injektion der Probe (Annahme: die Probe enthält 3 Komponenten A, B und C) wird die Probe auf der Trennsäule getrennt.

Während der Elution von Komponente A wird das Umschaltventil so geschaltet, daß $CO_2$ und Komponente A in Richtung des weiteren Umschaltventils umgeleitet wird. Das flüssige $CO_2$ wird entspannt. Das $CO_2$ verliert seine Lösungskraft. Komponente A wird dadurch auf der Fraktionierungssäule festgehalten. Das $CO_2$ wird durch einen Wärmetauscher weiter abgekühlt (falls notwendig) und gelangt wieder in den Vorratstank.

Nach Elution von Komponente A von der Säule wird das Umschaltventil auf eine weitere Fraktionierungssäule umgeschaltet. Die eluierende Komponente B wird auf dieser Fraktionierungssäule festgehalten. Komponente C auf einer weiteren Fraktionierungssäule. Eluiert keine Probesubstanz kann wahlweise der $CO_2$-Strom über eine der Fraktionierungssäulen geführt werden oder eine zusätzliche Rohrleitung mit einem Druckregelventil, welches das Umschaltventil mit dem weiteren Umschaltventil verbinden.

Das chromatographische Material der Trennsäule ist so gewählt, daß die Auflösung der Probekomponenten maximal ist. Das Material der Fraktionierungssäulen wird so gewählt, daß Adsorption der jeweiligen Probekomponente unter den dort herrschenden Bedingungen während der Trennung maximal ist.

Diese Vorgehensweise kann für mehrere Injektionen wiederholt werden (bis die Kapazität einer Fraktionierungssäule erreicht ist). Die Elution der jeweiligen Komponenten erfolgt mit einem geeigneten starken Lösemittel, z. B. Ethanol. Eine separate Pumpe fördert den Eluenten über das Umschaltventil bis zum Auslaß nach dem weiteren Umschaltventil. Die Wertfraktionen werden also in diesem Fall in Flüssigkeit gelöst aufgefangen.

Der Vorteil dieser beschriebenen Vorrichtung liegt darin, das weder ein vierter Wärmetauscher noch ein Abscheider benötigt wird.

In weiteren Ausgestaltungen des erfindungsgemäßen Verfahrens kann das Druckregelventil direkt hinter der Trennsäule angebracht sein. Das expandierte $CO_2$ mit der jeweiligen Probekomponente wird erst nach der Entspannung mittels des Umschaltventils auf die betreffende Fraktionierungssäule geleitet. Der Vorteil gegenüber der voranstehend beschriebenen Anordnung ist, daß nur ein Druckregelventil benötigt wird.

In weiteren Ausgestaltungen des erfindungsgemäßen Verfahrens können die Druckregelventile hinter den Fraktionierungssäulen angebracht sind. Zusätzlich befinden sich dahinter zusätzliche Abscheider.

Bei dieser Anordnung wird der Druck hinter der Fraktionierungssäule reguliert. Das Material der Fraktionierungssäulen wird so gewählt, daß die Komponenten A, B und C bei den herrschenden Bedingungen in der betreffenden Fraktionierungssäule zurückgehalten werden. Nach mehrmaliger Injektion und Sammeln der Probekomponenten auf den Fraktionierungssäulen können die einzelnen Komponenten durch Erhöhung der Elutionsstärke des $CO_2$ und/oder Zusatz eines Modifiziermittels zur Elution und über den jeweiligen Abscheider vom $CO_2$ getrennt werden.

Alternativ kann auch die Elution mit einem flüssigen Eluenten erfolgen. In diesem Fall können die Abscheider entfallen. Die Anordnung kann auch so abgewandelt werden, daß anstelle von drei Abscheidern nur ein Abscheider hinter dem Umschaltventil verwendet wird.

Durch das erfindungsgemäße Verfahren kann eine übliche SFC-Anlage energetisch viel günstiger betrieben werden. Im Wärmetauscher nach dem Vorratstank muß das $CO_2$ überhaupt nicht oder nur wenig erwärmt werden. Durch den geringeren Druck vor der Druckregeleinheit ist auch die Abkühlung durch die adiabatische Expansion geringer und damit muß dem Wärmetauscher nach der Trennsäule viel weniger Energie zugeführt werden. Prinzipiell kann das erfindungsgemäße Verfahren auf jeder SFC- Anlage ausgeführt werden.

Das erfindungsgemäße Verfahren ist ganz besonders zur säulenchromatographischen Trennung von ungesättigten, insbesondere mehrfach ungesättigten höheren Fettsäuren oder deren Derivaten, wie Estern oder Lipiden, vorzugsweise aus Naturölen, wie Pflanzenöl, Algenöl, Öl von Mikroorganismen oder Pilzen, sowie Fischöl und somit zur Gewinnung und Reinigung von SDA (Stearidonsäure, 18:4, n3), AA (Arachidonsäure, 20:4, n-6), ETA (Eicosatetraensäure, 20:4, n-3), EPA 25 (Eicosapentaensäure, 20:5, n-3), DPA (Docosapentaensäure, 22:5, n-3), DHA (Docosahexaensäure, 22:6, n-3). GLA (gamma-Linolensäure 18:3, n-6), ALA (alpha-Linolensäure 18:3, n-3), DPA (Docosapentaensäure, 22:5, n-6) geeignet.

Die nachfolgenden Beispiele dienen zur Erläuterung der Erfindung, ohne die Erfindung auf diese Beispiele zu begrenzen.

Beispiel 1:

Es wurde eine übliche SFC-Anordnung verwendet, wie sie weiter oben beschrieben wurde. Die verwendeten Säulen wiesen eine Länge von bis zu 170 cm auf. Die normal verwendeten Arbeitsdrücke betrugen in der Regel zwischen 120 und 350 bar. Diese Säulen können in der SFC eingesetzt werden, da die Druckabfälle auch über einer so langen Säule nur gering sind (normalerweise 20-50 bar). Durch die geringen Druckabfälle können auch höhere Flüsse, die in der SFC, im Gegensatz zur HPLC, nur zu einen geringen Effizienzverlust führen, eingesetzt werden. Als Eluent wurde Kohlendioxid verwendet. Die Arbeitsparameter sind in der nachfolgenden Tabelle 1 aufgelistet. Neben erfindungsgemäßem Einsatz von flüssigem Kohlendioxid wurde auch überkritisches Kohlendioxid eingesetzt.

Tabelle 1:

| Arbeitsparameter | | | | | | | | |
|---|---|---|---|---|---|---|---|---|
| Berechnung p | Säule: 10l | L (cm) | d(cm) | R(cm) | dp(μm) | | | Typ |
| | | 110 | 9 | 4,5 | 25 | | | |
| | ρ (g/ml) | η dyn.(Pa s) | η dyn.(cPoise) | F (kg/h) | F (ml/min) | | Δp (bar) | |
| **80 bar, 50°C** | 0,219 | 2,05E-05 | 0,02053 | 300 | 22.831,05 | | **21,6** | SFC |
| **100 bar, 50°C** | 0,3899 | 2,95E-05 | 0,02945 | 300 | 12.823,80 | | **17,4** | SFC |
| **200 bar, 50°C** | 0,784 | 6,80E-05 | 0,06797 | 300 | 6.377,55 | | **20,0** | SFC |
| **25°C, 150 bar** | 0,8771 | 8,63E-05 | 0,0863 | 300 | 5.700,60 | | **22,7** | SbFC |
| **0°C, 150 bar** | 1 | 1,23E-04 | 0,1234 | 300 | 5.000,00 | | **28,4** | SbFC |
| **0°C, 300 bar** | 1,055 | 1,47E-04 | 0,1466 | 300 | 4.739,34 | | **32,0** | SbFC |
| **0°C, 40 bar** | 0,9333 | 1,01E-04 | 0,1009 | 300 | 5.357,33 | | **24,9** | LFC |
| **10°C, 50 bar** | 0,8705 | 8,00E-05 | 0,08 | 300 | 5.743,83 | | **21,2** | LFC |
| **25°C, 70 bar** | 0,744 | 6.04E-05 | 0,06043 | 300 | 6.720,43 | | **18,7** | LFC |
| **MeOH, 20°C** | 0,79 | 5,84E-04 | 0,584 | 300 | 6.329,11 | | **170,4** | HPLC |
| **EtOH, 20°C** | 0,789 | 1,20E-03 | 1,201 | 300 | 6.337,14 | | **350,9** | HPLC |

Berechnung gemäß:

$$\Delta p = \frac{F \cdot \eta \cdot L \cdot 1000}{60 \cdot \pi \cdot r^2 \cdot dp^2}$$

mit

Δp    Druckabfall in [bar]
F      Volumenfluß des Eluenten in [ml/min]
L      Länge der Säule in [cm]
η      Viskosität in [cPoise];          1000 CPoise=1Pa s
R      Innenradius der Säule in [cm]
Dp    Teilchendurchmesser in [um]

Beispiel 2:

Es wurden Trennungen eines Fischölethylesters auf zwei verschiedenen stationären Phasen durchgeführt. Gewählt wurde jeweils sowohl überkritische Bedingungen als auch eine Temperatur/Fluß-Kombination aus dem erfindungsgemäßen Bereich. In der folgenden Tabelle 2 sind die Reinheit der EPA und/oder DHA der aufgefangenen Fraktionen miteinander verglichen.
Es wurde ein Fischöl unter verschiedenen Bedingungen getrennt: flüssige Phase (T=18°C, $p_n$=52 bar; F=300 kg/h); überkritische Phase: (T=46°C, $p_n$=105 bar; F=300 kg/h); stationäre Phase: Aminopropyl; mobile Phase: $CO_2$.]
In Tabelle 2 ist der Gehalt an EPA in % der jeweiligen Fraktion aufgetragen. Wie leicht zu erkennen, ist der Verlauf sehr ähnlich. Jedoch erfolgte die Trennung im flüssigen erfindungsgemäßen Bereich viel schneller.
Eine Reduzierung von ca. 75 auf nur 40 min war möglich. Die absolut erzielbare Reinheit war lediglich etwas geringer (anstatt 95% bei überkritischen Trennbedingungen auf ca. 90% im flüssigen Bereich). Legt man jedoch zur Ausbeuteberechnung die Fraktionen mit einer EPA-Reinheit über 80% zugrunde so entsprach diese im überkritischen Bereich 52,7% der injizierten Masse und im flüssigen Bereich 52,0%. Diese Zahlen verdeutlichen, daß die erzielten Trennungen sehr ähnlich sind.

Jedoch sind die im flüssigen Bereich notwendigen Anlagen wesentlich billiger, da sie nur für Drücke bis ca. 70 bar geeignet sein müssen und nicht bis zu 300 bar ausgelegt werden.

Tabelle 2:

| Gehalt an EPA in % bei Trennung mit flüssigem Kohlendioxid | | Gehalt an EPA in % bei Trennung mit überkritischem Kohlendioxid | |
|---|---|---|---|
| Trenndauer (min) | Gehalt der Fraktion an EPA (%) | Trenndauer (min) | Gehalt der Fraktion an EPA (%) |
| 12 | 1,4 | 21 | 2,03 |
| 12,5 | 0,18 | 23 | 0,99 |
| 13 | 0,12 | 25 | 0,29 |
| 13,5 | 0,25 | 28 | 0,54 |
| 14 | 0,19 | 30 | 3,01 |
| 14,5 | 0,23 | 32 | 13 |
| 15 | 0,53 | 33 | 14,23 |
| 15,5 | 3,61 | 34 | 14,35 |
| 16 | 13,94 | 35 | 18,63 |
| 16,5 | 27,28 | 36 | 25,5 |
| 17 | 44,65 | 37 | 36,16 |
| 17,5 | 64,79 | 38 | 54,23 |
| 18 | 73,59 | 39 | 76,62 |
| 18,5 | 79,96 | 40 | 91,08 |
| 19 | 84,14 | 41 | 93,96 |
| 19,5 | 86,69 | 42 | 94,78 |
| 20,5 | 89,07 | 43 | 95,18 |
| 21 | 89,64 | 44 | 95,44 |
| 21,5 | 89,45 | 45 | 95,31 |
| 22 | 88,05 | 46 | 95,25 |
| 22,5 | 87,01 | 47 | 94,34 |
| 23 | 85,76 | 48 | 93,67 |
| 23,5 | 84,1 | 49 | 93,03 |
| 24 | 82,16 | 50 | 92,34 |
| 24,5 | 80,53 | 51 | 91,7 |
| 25 | 78,8 | 52 | 91,24 |
| 25,5 | 76,97 | 53 | 90,67 |
| 26 | 75,63 | 54 | 89,42 |
| 26,5 | 74,09 | 56 | 86,79 |
| 27 | 73,21 | 59 | 79,34 |
| 28,5 | 69,27 | 62 | 61,96 |
| 32 | 59,55 | 67 | 14,71 |
| 34 | 51,7 | 121 | 1,95 |
| 37 | 26,52 | | |
| 40 | 17,67 | | |

In Tabelle 3 sind für die gleichen Trennungen die Reinheiten der Fraktionen an sowohl EPA als auch DHA aufgetragen. Auch hier ist ersichtlich, daß die Trennungen vergleichbar sind, jedoch die Trennung im flüssigen Bereich ist mit dieser Phase in der halben Zeit durchführbar.

Es wurde ein Fischöl unter verschiedenen Bedingungen getrennt: flüssige Phase (T=18°C, pn=52 bar; F=300 kg/h); überkritische Phase: (T=46°C, pn=105 bar; F=300 kg/h); Phase: Aminopropyl; mobile Phase: $CO_2$

Tabelle 3:

| Gehalt an EPA/DPA in % bei Trennung mit flüssigem Kohlendioxid | | Gehalt an EPA/DPA in % bei Trennung mit überkritischem Kohlendioxid | |
|---|---|---|---|
| Trenndauer (min) | Gehalt der Fraktion an EPA/DPA (%) | Trenndauer (min) | Gehalt der Fraktion an EPA/DPA (%) |
| 12 | 1,4/2,99 | 21 | 2,03/85,53 |
| 12,5 | 0,18/0,31 | 23 | 0,99/9,92 |
| 14 | 0,12/0,18 | 25 | 0,29/0,65 |
| 13,5 | 0,2510,32 | 28 | 0,54/0,27 |
| 14 | 0,1910,26 | 30 | 3,01/0,37 |
| 14,5 | 0,23/0,27 | 32 | 13/0,28 |
| 15 | 0,53/0,12 | 33 | 14,23/02, |
| 15,5 | 3,61/0,16 | 34 | 14,35/0,01 |
| 16 | 13,94/0,01 | 35 | 18,63/0,01 |
| 16,5 | 27,28/0,11 | 36 | 25,5/0,15 |
| 17 | 44,65/0,01 | 37 | 36,16/0,01 |
| 17,5 | 64,79/0,01 | 38 | 54,23/0,01 |
| 18 | 73,59/0,01 | 39 | 76,62/0,22 |
| 18,5 | 79,96/0,01 | 40 | 91,08/0,01 |
| 19 | 84,14/0,01 | 41 | 93,96/0,01 |
| 19,5 | 86,69/0,01 | 42 | 94,78/0,01 |
| 20,5 | 89,0710,32 | 43 | 95,18/0,13 |
| 21 | 89,64/0,75 | 44 | 95,44/0,01 |
| 21,5 | 89,45/1,41 | 45 | 95,31/0,27 |
| 22 | 88,05/2,58 | 46 | 95,25/0,35 |
| 22,5 | 87,01/3,72 | 47 | 94,34/0,44 |
| 23 | 85,76/5,15 | 48 | 93,67/0,58 |
| 23,5 | 84,1/6,66 | 49 | 93,03/0,72 |
| 24 | 82,16/8,14 | 50 | 92,34/0,93 |
| 24,5 | 80,53/10 | 51 | 91,7/1,2 |
| 25 | 78,8/11,66 | 52 | 91,24/1,48 |
| 25,5 | 76,97/13,17 | 53 | 90,67/1,88 |
| 26 | 75,63/14,81 | 54 | 89,42/2,43 |
| 26,5 | 74,09/16,12 | 56 | 86,79/3,65 |
| 27 | 73,21/17,31 | 59 | 79,34/8,53 |
| 28,5 | 69,27/21,06 | 62 | 61,96/21,54 |
| 32 | 59,55/28,9 | 67 | 14,71/62,52 |
| 34 | 51,7/35,27 | 121 | 1,95/81,27 |
| 37 | 26,52/54,38 | | |
| 40 | 17,67/62,17 | | |

In Tabellen 4 und 5 ist eine Trennung des gleichen Ausgangsmaterials auf einer anderen stationären Phase gezeigt. Auch hier ist ersichtlich, daß die Trennungen unter verschiedenen Bedingungen sehr ähnlich sind. Auf dieser stationären Phase eluieren die Substanzen jedoch später bei flüssigen Bedingungen. Die maximal erreichbare EPA-Reinheit ist auf dieser stationären Phase ca. 80%. Definiert man als Vergleich für die Massenausbeute eine EPA-Reinheit von mehr als 70% so ist die Ausbeute mit 36,2% bei flüssigen Bedingungen wesentlich höher als bei überkritischen Bedingungen mit nur 30,3%. Das stark ausgeprägte Tailing sowohl der EPA als auch der DHA ist vermutlich auf noch hochaktive Silanolgruppen zurückzuführen. Diese Phase ist für die Trennung weniger geeignet. Es konnte jedoch gezeigt werden, daß die Trennungen in verschiedenen Zustandsbereichen vergleichbar sind.

Es wurde ein Fischöl unter verschiedenen Bedingungen getrennt: flüssige Phase (T=18°C, pn=52 bar; F=300 kg/h); überkritische Phase: (T=46°C, pn=160 bar; F=300 kg/h); stationäre Phase: Kieselgel; mobile Phase: $CO_2$.

Tabelle 4:

| Gehalt an EPA in %<br>bei Trennung mit flüssigem Kohlendioxid | | Gehalt an EPA in %<br>bei Trennung mit überkritischem Kohlendioxid | |
|---|---|---|---|
| Trenndauer<br>(min) | Gehalt der Fraktion<br>an EPA (%) | Trenndauer<br>(min) | Gehalt der Fraktion<br>an EPA (%) |
| 20 | 13,02 | 12 | 9,11 |
| 21 | 20,05 | 13 | 4,15 |
| 23 | 34,82 | 14 | 10,06 |
| 24 | 47,25 | 15 | 21,46 |
| 25 | 62,5 | 15,5 | 29,13 |
| 26 | 71,44 | 16 | 40,14 |
| 26,5 | 74,5 | 16,5 | 54,65 |
| 27 | 76,22 | 17 | 63,67 |
| 27,5 | 77,39 | 17,5 | 68,77 |
| 28 | 78,59 | 18 | 73,71 |
| 29 | 79,85 | 19 | 78,4 |
| 30 | 80,05 | 19,5 | 80,8 |
| 31 | 79,39 | 20 | 81,48 |
| 32 | 76,37 | 21 | 80,31 |
| 33 | 74 | 22 | 76,77 |
| 34 | 70,8 | 23 | 72,49 |
| 35 | 68,48 | 24 | 69,63 |
| 36 | 66,17 | 25 | 66,77 |
| 37 | 64,6 | 26 | 64,32 |
| 38 | 64 | 27 | 66,69 |
| 39 | 62,62 | 28 | 60,73 |
| 40 | 61,82 | 29 | 59,48 |
| 41 | 60,99 | 30 | 58,03 |
| 42 | 60,62 | 32 | 54,65 |
| 44 | 59,23 | 34 | 51,4 |
| 46 | 57,65 | 36 | 46,5 |
| 48 | 56,66 | 38 | 42,34 |
| 50 | 55,32 | 40 | 37,86 |
| 52 | 53,51 | 45 | 27,81 |
| 55 | 48,97 | | |
| 60 | 44,15 | | |

Tabelle 5:

| Gehalt an EPA/DMA in %<br>bei Trennung mit flüssigem Kohlendioxid | | Gehalt an EPA/DHA in %<br>bei Trennung mit überkritischem Kohlendioxid | |
|---|---|---|---|
| Trenndauer<br>(min) | Gehalt der Fraktion<br>an EPA/DHA (%) | Trenndauer<br>(min) | Gehalt der Fraktion<br>an EPA/DHA (%) |
| 20 | 13,02/1,69 | 12 | 9,11/26,52 |
| 21 | 20,05/0,92 | 13 | 4,15/3,59 |
| 23 | 34,82/0,48 | 14 | 10,06/1,12 |
| 24 | 47,25/0,65 | 15 | 21,46/0,45 |
| 25 | 62,5/0,92 | 15,5 | 29,13/0,41 |
| 26 | 71,44/1,38 | 16 | 40,14/0,39 |
| 26,5 | 74,5/1,63 | 16,5 | 54,65/0,52 |

Tabelle 5: (fortgesetzt)

| Gehalt an EPA/DMA in %<br>bei Trennung mit flüssigem Kohlendioxid | | Gehalt an EPA/DHA in %<br>bei Trennung mit überkritischem Kohlendioxid | |
|---|---|---|---|
| Trenndauer<br>(min) | Gehalt der Fraktion<br>an EPA/DHA (%) | Trenndauer<br>(min) | Gehalt der Fraktion<br>an EPA/DHA (%) |
| 27 | 76,22/1,93 | 17 | 63,67/0,73 |
| 27,5 | 77,39/2,19 | 17,5 | 68,77/0,97 |
| 28 | 78,59/2,54 | 18 | 73,71/1,43 |
| 29 | 79,85/3,42 | 19 | 78,4/2,23 |
| 30 | 80,05/4,59 | 19,5 | 80,8/2,68 |
| 31 | 79,39/6,21 | 20 | 81,48/3,21 |
| 32 | 76,37/8,36 | 21 | 80,31/5,23 |
| 33 | 74/11,88 | 22 | 76,77/8,96 |
| 34 | 70,8/15,08 | 23 | 72,49/13,52 |
| 35 | 68,48/17,73 | 24 | 69,63/16,38 |
| 36 | 66,17/19,53 | 25 | 66,77/19,32 |
| 37 | 64,6/21,21 | 26 | 64,32/22,11 |
| 38 | 64/22,2 | 27 | 66,69/20,21 |
| 39 | 62,62/23,39 | 28 | 60,73/25,81 |
| 40 | 61,82/24,35 | 29 | 59,48/ 27,33 |
| 41 | 60,99/24,8 | 30 | 58,03/28,62 |
| 42 | 60,62/25,12 | 32 | 54,65/31,5 |
| 44 | 59,23/26,89 | 34 | 51,4/34,19 |
| 46 | 57,65/28,31 | 36 | 46,5/38,47 |
| 48 | 56,66/29,39 | 38 | 42,34/42,23 |
| 50 | 55,32/30,58 | 40 | 37,86/45,76 |
| 52 | 53,51/31,95 | 45 | 27,81/53,76 |
| 55 | 48,97/34,7 | | |
| 60 | 44,15/38,6 | | |

Im Text verwendete Abkürzungen:

HPLC     Hochleistungsflüssigkeitschromatographie
SFC     Chromatographie mit überkritischem Gas
SbFC     Chromatographie mit fluidem Gas ($T<T_{kr}$; $p>p_{kr}$)
LFC     Chromatographie mit flüssigem Gas ($T<T_{kr}$; $p<p_{kr}$)
EPA     Eicosapentaensäurethylester (20:5)
DHA     Docosahexaensäure (22:6)

**Patentansprüche**

**1.** Verfahren zur säulenchromatographischen Trennung von Stoffgemischen, wobei als Eluent ein Fluid oder eine Mischung von Fluiden in flüssigem, nicht-subkritischem und nicht-kritischem Zustand ($T<T_{kr}$ und $p<p_{kr}$) zum Einsatz kommt, die bei 25°C und 1 bar gasförmig sind.

**2.** Verfahren nach Anspruch 1, **dadurch gekennzeichnet, daß** der Eluent ausgewählt wird aus der Gruppe bestehend aus flüssigem Distickstoffoxid, flüssigem Fluorkohlenwasserstoff oder Fluorchlorkohlenwasserstoff, flüssigem Kohlendioxid, flüssigem Schwefelhexafluorid, flüssigem Propen, flüssigem Propan, flüssigem Ammoniak, flüssigem Schwefeldioxid, flüssigem Xenon. flussigem Ethan oder einer Mischung davon.

**3.** Verfahren nach Anspruch 1, **dadurch gekennzeichnet, daß** der Eluent eine dynamische Viskosität von $10^{-4}$ - $10^{-5}$ Pa s, vorzugsweise $2*10^{-4}$ - $2*10^{-5}$ Pa s, eine Dichte von 0,5 - 1,2 g/ml, vorzugswelse von 0,5-1,2 g/ml aufweist

**4.** Verfahren nach Anspruch 1, **dadurch gekennzeichnet, daß** der Eluent flüssiges Kohlendioxid ist, vorzugsweise in einem Temperaturbereich von 0 bis 20°C.

**5.** Verfahren nach Anspruch 1, **dadurch gekennzeichnet, daß** der Eluent flüssiges Kohlendioxid ist in einem Druckbereich von 30 bis 73,7 bar.

**6.** Verfahren nach Anspruch 1, **dadurch gekennzeichnet, daß** der Eluent mindestens ein Modifiziermittel enthält.

**7.** Verfahren nach Anspruch 1, **dadurch gekennzeichnet, daß** eine Säulenlänge von mindestens 10 cm verwendet wird.

**8.** Verfahren nach Anspruch 7, **dadurch gekennzeichnet, daß** eine Säulenlänge von 0,25 bis 2,0 m verwendet wird.

**9.** Verfahren nach Anspruch 8, **dadurch gekennzeichnet, daß** eine Säutenlänge von 1,1 bis 1,7 m verwendet wird.

**10.** Verfahren nach Anspruch 1, **dadurch gekennzeichnet, daß** als stationäre Phase modifizierte oder unmodifizierte Phasen ausgewählt aus der Gruppe bestehend aus Kieselgel, Aluminiumoxid. Titandioxid sowie geträgerte polymere stationäre Phasen verwendet werden.

**11.** Verfahren nach Anspruch 10, **dadurch gekennzeichnet, daß** die stationäre modifizierte Phase ausgewählt wird aus der Gruppe bestehend aus auf Kieselgel, Aluminiumoxid oder Titandioxid aufgebrachte Diolphase, Aminopropylphase, RP8 und RP18 Phase.

**12.** Verfahren nach Anspruch 1, **dadurch gekennzeichnet, daß** als Stoffgemische Naturöle enthaltend ungesättigte Fettsäuren verwendet werden.

**13.** Verwendung von Fluiden oder einer Mischung von Fluiden in flüssigem, nicht-subkritischem und nicht-kritischem Zustand (T < $T_{kr}$ und p < $p_{kr}$) die bei 25°C und 1 bar gasförmig sind, zur Isolierung und / oder Reinigung und/oder präparativen Gewinnung von Stoffgemischen mittels Säulenchromatographie.

**Claims**

**1.** Method for the column-chromatographic separation of mixtures of materials using a fluid or a mixture of fluids which are gaseous at 25°C and 1 bar in a liquid, nonsubcritical and noncritical state (T<$T_{cr}$ and P<$P_{cr}$) as eluant.

**2.** Method according to Claim 1, **characterized in that** the eluant is selected from the group consisting of liquid dinitrogen oxide, liquid fluorinated hydrocarbons and chlorofluorocarbons, liquid carbon dioxide, liquid sulphur hexafluoride, liquid propene, liquid propane, liquid ammonia, liquid sulphur dioxide, liquid xenon, liquid ethane and mixtures thereof.

**3.** Method according to Claim 1, **characterized in that** the eluant has a dynamic viscosity of $10^{-4}$ - $10^{-5}$ Pa s, preferably $2*10^{-4}$ - $2*10^{-5}$ Pa s, a density of 0.5 - 1.2 g/ml, preferably 0.5 - 1.2 g/ml.

**4.** Method according to Claim 1, **characterized in that** the eluant is liquid carbon dioxide, preferably in a temperature range from 0 to 20°C.

**5.** Method according to Claim 1, **characterized in that** the eluant is liquid carbon dioxide in a pressure range from 30 to 73.7 bar.

**6.** Method according to Claim 1, **characterized in that** the eluant comprises at least one modifier.

**7.** Method according to Claim 1, **characterized in that** a column length of at least 10 cm is used.

**8.** Method according to Claim 7, **characterized in that** a column length of from 0.25 to 2.0 m is used.

**9.** Method according to Claim 8, **characterized in that** a column length of from 1.1 to 1.7 m is used.

**10.** Method according to Claim 1, **characterized in that** the stationary phase used is a modified or unmodified phase selected from the group consisting of silica gel, aluminium oxide, titanium dioxide and supported polymeric stationary phases.

**11.** Method according to Claim 10, **characterized in that** the stationary modified phase is selected from the group consisting of a diol phase, aminopropyl phase, RP8 and RP18 phase applied to silica gel, aluminium oxide or titanium dioxide.

**12.** Method according to Claim 1, **characterized in that** the mixtures of materials used are natural oils comprising unsaturated fatty acids.

**13.** Use of fluids or a mixture of fluids which are gaseous at 25°C and 1 bar in the liquid, nonsubcritical and noncritical state ($T<T_{cr}$ and $p<p_{cr}$) for the isolation and/or purification and/or preparative recovery of mixtures of materials by means of column chromatography.

## Revendications

**1.** Procédé de séparation de mélanges de substances par chromatographie sur colonne dans lequel on utilise comme éluant un fluide ou un mélange de fluides à l'état liquide, non sous-critique et non critique ($T < T_{cr}$ et $p < p_{cr}$), qui sont gazeux à 25°C et 1 bar.

**2.** Procédé selon la revendication 1, **caractérisé en ce que** l'éluant est choisi dans le groupe constitué de l'oxyde de diazote liquide, un fluorohydrocarbure ou un fluorochlorohydrocarbure liquide, le dioxyde de carbone liquide, l'hexafluorure de soufre liquide, le propène liquide, le propane liquide, l'ammoniac liquide, le dioxyde de soufre liquide, le xénon liquide, l'éthane liquide ou un mélange de ceux-ci.

**3.** Procédé selon la revendication 1, **caractérisé en ce que** l'éluant présente une viscosité dynamique de $10^{-4}$ à $10^{-5}$ Pa·s, de préférence de $2·10^{-4}$ à $2·10^{-5}$ Pa·s, une masse volumique de 0,5 à 1,2 g/ml, de préférence de 0,5 à 1,2 g/ml.

**4.** Procédé selon la revendication 1, **caractérisé en ce que** l'éluant est le dioxyde de carbone liquide, de préférence dans une gamme de température de 0 à 20°C.

**5.** Procédé selon la revendication 1, **caractérisé en ce que** l'éluant est le dioxyde de carbone liquide dans une gamme de pression de 30 à 73,7 bar.

**6.** Procédé selon la revendication 1, **caractérisé en ce que** l'éluant contient au moins un agent modifiant.

**7.** Procédé selon la revendication 1, **caractérisé en ce qu'**on utilise une longueur de colonne d'au moins 10 cm.

**8.** Procédé selon la revendication 7, **caractérisé en ce qu'**on utilise une longueur de colonne de 0,25 à 2,0 m.

**9.** Procédé selon la revendication 8, **caractérisé en ce qu'**on utilise une longueur de colonne de 1,1 à 1,7 m.

**10.** Procédé selon la revendication 1, **caractérisé en ce qu'**on utilise comme phase stationnaire des phases modifiées ou non modifiées choisies dans le groupe constitué du gel de silice, de l'oxyde d'aluminium, du dioxyde de titane ainsi que des phases stationnaires de polymères supportés.

**11.** Procédé selon la revendication 10, **caractérisé en ce que** la phase stationnaire modifiée est choisie dans le groupe constitué d'une phase diol, d'une phase aminopropyle, d'une phase RP8 et RP18 appliquée sur gel de silice, oxyde d'aluminium ou dioxyde de titane.

**12.** Procédé selon la revendication 1, **caractérisé en ce qu'**on utilise comme mélanges de substances des acides gras insaturés contenant des huiles naturelles.

**13.** Utilisation de fluides ou d'un mélange de fluides à l'état à l'état liquide, non sous-critique et non critique ($T < T_{cr}$ et $p < P_{cr}$), qui sont gazeux à 25°C et 1 bar, pour l'isolement et/ou la purification et/ou l'obtention préparative de mélanges de substances au moyen au moyen de la chromatographie sur colonne.